(19) Europäisches Patentamt
European Patent Office
Office européen des brevets

(11) Publication number: **0 302 934 A1**

(12) # EUROPEAN PATENT APPLICATION

published in accordance with Art. 158(3) EPC

(21) Application number: **87901653.3**

(22) Date of filing: **26.02.87**

Data of the international application taken as a basis:

(86) International application number: **PCT/JP87/00121**

(87) International publication number: **WO88/06581 (07.09.88 88/20)**

(51) Int. Cl.[3]: **C 07 C 103/737, C 07 D 209/48, A 61 K 31/19, A 61 K 31/40**

(43) Date of publication of application: **15.02.89 Bulletin 89/7**

(84) Designated Contracting States: **BE CH DE FR GB IT LI LU NL SE**

(71) Applicant: **NIPPON ZEON CO., LTD.**
**6-1, Marunouchi 2-chome**
**Chiyoda-ku Tokyo 100(JP)**

(71) Applicant: **TAISHO PHARMACEUTICAL CO. LTD**
**24-1 Takata 3-chome Toshima-ku**
**Tokyo 171(JP)**

(72) Inventor: **ONAKA, Michihiro**
**5-3-3, Inamuragasaki**
**Kamakura-shi Kanagawa 248(JP)**

(72) Inventor: **MATSUNO, Shugo**
**2-26-3-104, Kajiwara**
**Kamakura-shi Kanagawa 247(JP)**

(72) Inventor: **MOCHIZUKI, Naoki**
**1-42-1, Moto-Ohashi**
**Sakae-ku, Yokohama-shi Kanagawa 247(JP)**

(72) Inventor: **KURACHI, Michio**
**94-7, Motojuku 6-chome**
**Kitamoto-shi Saitama 364(JP)**

(72) Inventor: **OTOMO, Susumu**
**785-341, Kamioinezuka**
**Konosu-shi Saitama 365(JP)**

(72) Inventor: **AIHARA, Hironaka**
**1514-40, Kitanakamaru**
**Kitamoto-shi Saitama 364(JP)**

(74) Representative: **Harrison, David Christopher et al,**
**MEWBURN ELLIS & CO 2/3 Cursitor Street**
**London EC4A 1BQ(GB)**

(54) **ALICYCLIC DICARBOXYLIC ACID DERIVATIVES AND MUSCLE RELAXANTS CONTAINING THEM AS EFFECTIVE INGREDIENTS.**

(57) Alicyclic dicarboxylic acid derivatives represented by general formula (I),

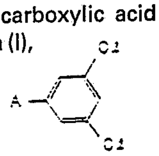
(I)

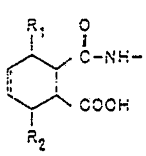
(II)

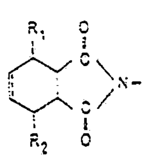
(III)

(wherein A represents (II) or (III), $R_1$ and $R_2$ in (II) or (III) each represents a hydrogen atom or a lower alklyl group, with at least one of $R_1$ and $R_2$ being a lower alkyl group, and ---- represents a single or double bond) and salts thereof, and muscle relaxants containing said alicyclic dicarboxylic acid derivatives or pharmacologically acceptable salts thereof as effective ingredients are disclosed. The drugs are effective in treating spastic paralysis, lumbodorsalgia, herniation of the intervertebral disk, spondylolysis, osteoporosis, neck-shoulder-arm syndrome, etc.

EP 0 302 934 A1

SPECIFICATION

# ALICYCLIC DICARBOXYLIC ACID DERIVATIVES AND MUSCLE RELAXANT CONTAINING THE SAME AS ACTIVE INGREDIENT

## TECHNICAL FIELD OF THE INVENTION

This invention relates to novel alicyclic dicarboxylic acid derivatives, as well as to a muscle relaxant for mammals and particularly for human being containing the same as active ingredient.

## BACKGROUND OF THE INVENTION

A number of alicyclic dicarboxylic acid derivatives obtainable by reacting an alicyclic dicarboxylic anhydride such as hexahydrophthalic anhydride, tetrahydrophthalic anhydride and the like with various aniline compounds have been disclosed until today (for example, Japanese Patent Publication No. 47-19,196, Japanese Patent Application Kokai (Laid-Open) No. 59-231,057, etc.). However, the use of the hitherto known alicyclic dicarboxylic acid derivatives is limited to agricultural fungicide and therapeutic agent for convulsive diseases such as epilepsy, and it has been entirely unknown whether these compounds have a muscle-relaxant action or not.

## DISCLOSURE OF THE INVENTION

The present inventors conducted many studies on the analogues of such alicyclic dicarboxylic acid

derivatives. As the result, it was found that, among the alicyclic dicarboxylic acid derivatives, the novel compounds represented by the general formula (I) mentioned later, not found in literature, specifically exhibit a marked muscle-relaxant action on test animals. Based on this finding, this invention was accomplished.

According to this invention, there is provided, as the first invention, an alicyclic dicarboxylic acid derivative represented by the following general formula (I) and salts thereof:

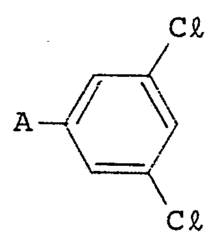

(I)

wherein A represents a group represented by general formula (II) or general formula (III):

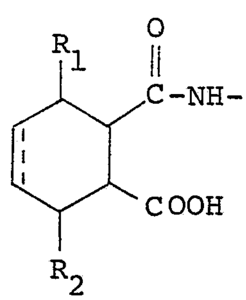

(II)

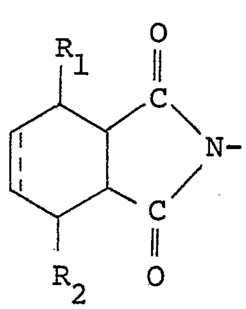

(III)

($R_1$ and $R_2$ in (II) and (III) represent a hydrogen atom or a lower alkyl group, provided that at least one of $R_1$ and $R_2$ represents a lower alkyl group, and $\equiv$ represents a single bond or a double bond); and there is provided, as the second invention, a muscle relaxant composition containing these compounds as active ingredient.

BEST EMBODIMENT FOR PRACTICE OF THE INVENTION

Hereunder, the content of this invention will be mentioned in detail.

The compounds represented by general formula (I) can be synthesized by various methods. Among these methods, an example of the usually employed method will be mentioned below.

For example, when A in general formula (I) is a group represented by general formula (II), the objective compound (I) can be obtained by reacting 3,5-dichloroaniline represented by the following general formula (IV) with an acid anhydride represented by the following general formula (V) in the usual manner to form an amide:

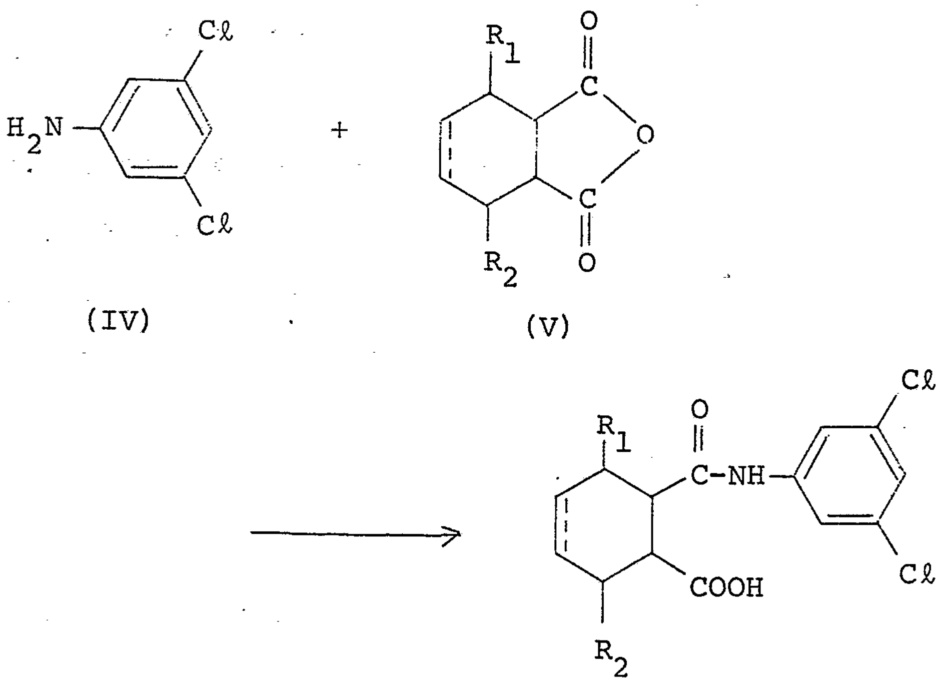

(IV) (V)

(I)

In this reaction, the presence of solvent is not indispensable. However, the use of solvent is preferable from the viewpoint of efficiency of reaction. Examples of preferable solvent include aromatic liquid compounds such as benzene, toluene and the like, halogenated hydrocarbons such as chloroform, chlorobenzene and the like, and ketones such as acetone, methyl ethyl ketone and the like.

For synthesizing a salt of product (I), a carboxylic acid amide compound prepared according to the above-mentioned reaction is neutralized according to the conventional method. As the cation for forming the salt, any cations may be used so far as they are pharmacologically acceptable. For example, alkali metal ions such as sodium ion, potassium ion and the like, alkaline earth metal ions such as calcium ion, magnesium ion and

the like, ammonium ion, etc. can be used for this purpose.

When A in general formula (I) is a group represented by general formula (III), the compound (I) can be obtained by imidating the carboxylic acid amide compound obtained by the above-mentioned method either by a heat treatment or by the use of an acid catalyst such as p-toluenesulfonic acid, sulfuric acid, hydrochloric acid or the like, a basic catalyst such as sodium acetate, triethylamine or the like, or a dehydrating catalyst such as acetic anhydride, thionyl chloride or the like. In this reaction, the reaction temperature is usually 20°C to 250°C and preferably 50°C to 150°C; and the reaction time is usually 10 minutes to 20 hours and preferably 30 minutes to 5 hours.

If a lower fatty acid such as acetic acid, propionic acid or the like is used in the above-mentioned reaction in place of the solvent, an imide compound can be obtained directly without passing through the amide.

As shown in the aforementioned formulas (I), (II) and (III), the characteristic feature of the alicyclic dicarboxylic acid derivatives obtained according to the invention consists in the position of the lower alkyl group linked to the cyclohexane ring or cyclohexene ring and the position of the chlorine atom on the aromatic ring.

As concrete examples of the lower alkyl group in formulas (II) and (III), methyl, ethyl and the like

can be referred to, among which methyl group is preferred from the viewpoint of efficacy.

Typical examples of the compound represented by general formula (I) include the followings:

2-(3',5'-Dichlorophenylcarbamoyl)-3-methyl-cyclohexanecarboxylic acid,

2-(3',5'-Dichlorophenylcarbamoyl)-6-methyl-cyclohexanecarboxylic acid,

2-(3',5'-Dichlorophenylcarbamoyl)-3,6-dimethylcyclohexanecarboxylic acid,

2-(3',5'-Dichlorophenylcarbamoyl)-3-ethyl-cyclohexanecarboxylic acid,

2-(3',5'-Dichlorophenylcarbamoyl)-6-ethyl-cyclohexanecarboxylic acid,

2-(3',5'-Dichlorophenylcarbamoyl)-3-methyl-4-cyclohexenecarboxylic acid,

2-(3',5'-Dichlorophenylcarbamoyl)-6-methyl-4-cyclohexenecarboxylic acid,

2-(3',5'-Dichlorophenylcarbamoyl)-3,6-dimethyl-4-cyclohexenecarboxylic acid,

2-(3',5'-Dichlorophenylcarbamoyl)-3-ethyl-4-cyclohexenecarboxylic acid,

2-(3',5'-Dichlorophenylcarbamoyl)6-ethyl-4-cyclohexenecarboxylic acid,

Sodium salts, potassium salt, calcium salts, magnesium salts and ammonium salts of these compounds,

N-(3',5'-Dichlorophenyl)-3-methylcyclohexane-1,2-dicarboxylic acid imide,

N-(3',5'-Dichlorophenyl)-3,6-dimethylcyclohexane-1,2-dicarboxylic acid imide,

N-(3',5'-Dichlorophenyl)-3-ethylcyclohexane-1,2-dicarboxylic acid imide,

N-(3',5'-Dichlorophenyl)-3-methyl-4-cyclohexene-1,2-dicarboxylic acid imide,

N-(3',5'-Dichlorophenyl)-3,6-dimethyl-4-cyclohexene-1,2-dicarboxylic acid imide,

N-(3',5'-Dichlorophenyl)-3-ethyl-4-cyclohexene-1,2-dicarboxylic acid imide.

The compound represented by general formula (I) exercises only a very low toxicity on human and mammals. The oral acute toxicity ($LD_{50}$) on male mouse is generally lower than 1,000 mg/kg body weight.

This compound exhibits a marked muscle relaxing effect on test animals, and is useful as a therapeutic agent for spastic paralysis caused by the disturbance of cerebral blood vessels, the spastic spinal paralysis, the cervical spondylosis, etc. and for painful contractures caused by the pain of back and low back, hernia of intervertebral disc, spondylolysis, spondylolisthesis, spinal osteoporosis, cervical sydorome, etc.

The compound of this invention may be administered to patients having the above-mentioned diseases by any of the oral administration and parenteral administration. Its dose may be appropriately decided in accordance with kind of disease, extent of symptoms,

age of patient, body weight of patient, etc. For example, in case of oral administration to adult, it is administered at a rate of 0.1 to 30 mg/kg-body weight/day. In case of intravenous administration to adult, it is administered at a rate of 5 to 1,000 μg/kg-body weight/day. In both the cases, the daily dose is given in one to three portions everyday.

The compound of this invention can be made into a composition form suitable for the route of administration. For example, for oral administration, tablet, granule, capsule and powder compositions can be adopted. For the parenteral administration, solution, suspension and emulsion compositions can be adopted. These compositions can be prepared by mixing an effective amount of the compound of this invention with pharmaceutically acceptable carrier and diluent in the conventional manner.

The carriers which can be used for preparing oral solid composition include vehicles such as lactose, glucose, crystalline cellulose, mannitol, corn starch, sucrose and the like, binders such as hydroxypropyl cellulose, hydroxypropyl methyl cellulose, carboxymethyl cellulose, polyvinyl alcohol, gelatin, gum arabic and the like, wetting agents such as glycerin, ethylene glycol and the like, disintegrators such as corn starch, potato starch, carboxymethyl cellulose calcium, hydroxypropyl cellulose of low degree of substitution, and the like, and lubricants such as calcium stearate, magnesium

stearate, talc, polyethylene glycol, hydrogenated oil and the like. If desired, surfactant, colorant, flavoring agent and the like may also be used in addition to above.

The diluents which can be used in the preparation of oral liquid composition include water, ethanol, glycerin, propylene glycol, polyethylene glycol, agar, Tragacantha and the like. If desired, dissolution aid, buffer, preservative, perfume, colorant, flavoring agent and the like may also be used.

Examples of the carrier which can be used in the preparation of parenteral compositions such as injection include purified water for injection, naturally occurring vegetable oils such as soybean oil, synthetic fatty acid esters such as oleic acid glyceride and/or emulsifiers such as phospholipid. If desired, pH buffering agent, isotonizing agent, antiseptic, antioxidant and the like may also be added to the composition.

Next, this invention will be illustrated more concretely by way of the following synthesis examples of the compound of general formula (I), examples of this invention and test examples.

Synthesis Example 1

Synthesis of N-(3',5'-Dichlorophenyl)-3-methyl-4-cyclohexene-1,2-dicarboxylic Acid Imide

In 100 ml of acetic acid, 9.8 g of 3-methyl-1,2,3,6-tetrahydrophthalic anhydride and 9.6 g of

3,5-dichloroaniline were reacted under reflux for 4 hours. After cooling the reaction mixture, water was added, and the resulting crude crystal was collected by filtration. Then, it was recrystallized from ethanol to obtain 10 g of the objective compound (hereinafter, this is referred to as "Compound 1").

Yield 55%; m.p. 95-96°C

Synthesis Examples 2 to 4

Compounds 2 to 4 were synthesized in the same manner as in Synthesis Example 1, except that the 3-methyl-1,2,3,6-tetrahydrophthalic anhydride was replaced with 3-ethyl-1,2,3,6-tetrahydrophthalic anhydride in the synthesis of Compound 2, with 3,6-dimethyl-1,2,3,6-tetrahydrophthalic anhydride in the synthesis of Compound 3, and with 3,6-dimethylhexahydrophthalic anhydride in the synthesis of Compound 4. The results are shown in Table 1.

Table 1

| Ex. No. | Structural formula | m.p. (°C) |
|---|---|---|
| 2 | 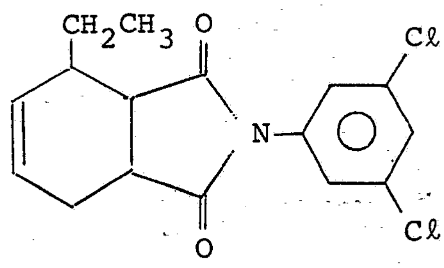 | 91 ∿ 92 |
| 3 | 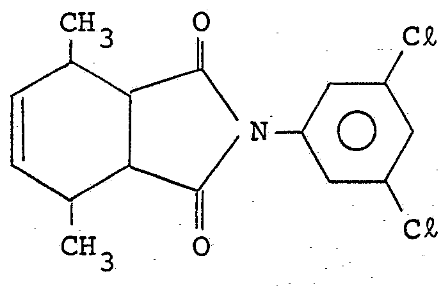 | 81 ∿ 82 |
| 4 | 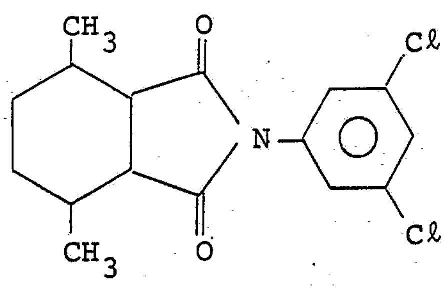 | Pasty * |

* $^{1}H$-NMR ($CDC\ell_3$, TMS) ppm;

1.2 (6H, d), 1.5 ∿ 1.9 (4H, m), 2.3 (2H, m),

3.1 (2H, m), 7.0 ∿ 7.3 (3H, m)

Synthesis Example 5

Synthesis of 2-(3',5'-Dichlorophenylcarbamoyl)-3(6)-methyl-4-cyclohexenecarboxylic Acid

In 100 ml of benzene, 9.8 g of 3-methyl-1,2,3,6-tetrahydrophthalic anhydride and 9.6 g of 3,5-dichloroaniline were stirred at room temperature. Collection of the resulting crystal by filtration gave 15 g of the objective product (hereinafter referred to as "Compound 5") was obtained.

Yield 78%; m.p. 149-151°C

Synthesis Example 6

Synthesis of 2-(3',5'-Dichlorophenylcarbamoyl)-3,6-dimethyl-4-cyclohexenecarboxylic Acid

The procedure of Synthesis Example 5 was repeated, except that 3,6-dimethyl-1,2,3,6-tetrahydrophthalic anhydride and 3,5-dichloroaniline were used as starting compounds. As the result, the objective product (hereinafter referred to as "Compound 6") was obtained.

m.p. 176-177°C

Synthesis Example 7

Synthesis of Sodium 2-(3',5'-Dichlorophenylcarbamoyl)-3,6-dimethyl-4-cyclohexenecarboxylate

Compound 6 (3.4 g) was dissolved in 100 ml of 0.1 N methanolic solution of sodium hydroxide. By distilling off the solvent, there was obtained 3.4 g of

the objective product (hereinafter referred to as "Compound 7").

Elementary analyses for $C_{16}H_{16}NO_3Cl_2Na$

| | C | H | N |
|---|---|---|---|
| Calculated (%) | 52.77 | 4.43 | 3.85 |
| Found (%) | 52.65 | 4.52 | 3.79 |

Example 1

A granular composition was prepared by mixing 600 g of Compound 3, 120 g of crystalline cellulose and 126 g of corn starch to prepare a uniform powdery mixture, adding thereto 45 g of hydroxypropyl cellulose as a binder, and forming the mixture by the wet granulating process.

After adding 9 g of magnesium stearate to the granule, the mixture was tableted to obtain 3,000 tablets having a diameter of 9 mm and a weight of 300 mg/tablet.

Example 2

A uniform mixture was prepared from 600 g of Compound 1, 150 g of crystalline cellulose, 140 g of corn starch and 10 g of magnesium stearate.

This powdery mixture was divided into 300 mg portions, and each portion was packed into No. 1 hard capsule. Thus, 3,000 capsules were obtained.

Example 3

A uniform powdery mixture was prepared by

blending 200 g of Compound 5, 300 g of mannitol and 450 g of corn starch. After adding 50 g of hydroxypropyl cellulose as a binder, the composition was formed into granules by the wet granulating process. Thus, a granular composition was obtained.

Example 4

A powdery composition was prepared by uniformly mixing 200 g of Compound 7 and 800 g of lactose. The mixture was divided into 1,000 mg portions, and each portion was wrapped. Thus, 1,000 wrappages of powdery composition was obtained.

Test Example 1

(1) Horizontal wire test

Each of the Compounds 1, 3, 4, 5 and 7 obtained in the synthesis examples was suspended into 0.4% aqueous solution of carboxymethyl cellulose to prepare test drugs having a concentration of 1%. Each test drug was administered orally to respective group of test animals (ICR male mice, body weight 20 to 30 g, 6 animals per one group), so that the amount of active ingredient administered came to 100 mg/kg-body weight. Thereafter, at constant intervals of time, the mice were suspended by their forepaws on the metallic wire held horizontally, and the number of animals failing to hang was counted. The maximum numbers of fallen mice are summarized in Table 2.

Table 2

| Test No. | Test group (administered compound) | No. of cases | No. of fallen cases |
|---|---|---|---|
| 1-1 | Compound 1 | 6 | 5 |
| 1-2 | Compound 3 | 6 | 3 |
| 1-3 | Compound 4 | 6 | 3 |
| 1-4 | Compound 5 | 6 | 3 |
| 1-5 | Compound 7 | 6 | 3 |

(2) Test method using anaemic decerebrate rigidity animals

Each of Compound 1 and Compound 3 obtained in the synthesis examples was dissolved into 100% polyethylene glycol (molecular weight ca. 400) to prepare test drugs having a concentration of 0.3%. According to the method of Fukuda et al. (Fukuda, H., Ito, T., Hashimoto, S. and Kudo, Y., Japan. J. Pharmacol., (1974) 24; 810), the rigidity was induced in Wister male rats (body weight 250 to 350 g). Thus, by ligating both the common carotid arteries and coagulating the basilarartery by means of high frequency wave, the rigidity was induced in the fore legs of the animals. Using three animals as one group, each test drug was intravenously administered to the animals so that the amount of active ingredient came to 3 mg/kg body weight. Thereafter, whether the rigidity in the fore legs of the rat was relaxed or not was visually followed over

a continuous 60 minutes. As the result, the rigidity disappeared in all the groups.

Among the groups tested, the group to which Compound 3 was administered exhibited a particularly marked rigidity - eliminating effect.

Test Example 2

(1) Acute toxicity test

ICR male mice (body weight 20 to 30 g, 10 animals per one group) were used for the test.

A test drug was prepared by suspending Compound 3 into 0.4% aqueous solution of carboxymethyl cellulose, and it was orally administered.

Thereafter, the state of the animals was visually observed for 5 days. As the result, $LD_{50}$ (mg/kg) was 1,000 mg/kg or more.

INDUSTRIAL UTILIZABILITY

The compounds of this invention represented by general formula (I) and their salts are effectively usable for the therapy of spastic paralysis, the pain of back and low back, hernia of intervertebral disc, spondylolysis, spondylolisthesis, spinal osteoporosis, cervical syndorome, etc. of human being and mammals provoked by various causes, and it can be used as a muscle relaxant. Accordingly, this invention has a great industrial meaning.

WHAT IS CLAIMED IS:

1. An alicyclic dicarboxylic acid derivative represented by the following general formula (I) and a salt thereof:

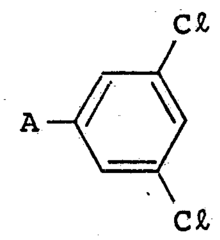

(I)

wherein A is a group represented by the following general formula (II):

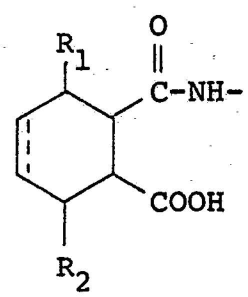

(II)

or general formula (III):

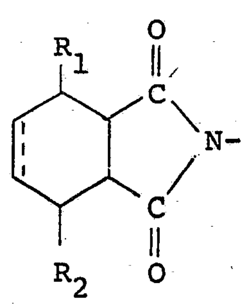

(III)

(in formulas (II) and (III), $R_1$ and $R_2$ represent a hydrogen atom or a lower alkyl group, provided that at least one of $R_1$ and $R_2$ represents a lower alkyl group, and ═══ represents a single bond or a double bond).

2. An alicyclic dicarboxylic acid and a salt

thereof according to Claim 1, wherein $R_1$ and $R_2$ of general formulas (II) and (III) both represents a lower alkyl group.

3. An alicyclic dicarboxylic acid and a salt thereof according to Claim 2, wherein said lower alkyl group is a methyl group or an ethyl group.

4. An alicyclic dicarboxylic acid and a salt thereof according to Claim 2, wherein said lower alkyl group is a methyl group.

5. An alicyclic dicarboxylic acid and a salt thereof according to Claim 1, wherein said salt is a salt of alkali metal, alkaline earth metal or ammonium.

6. A muscle relaxant comprising, as its active ingredient, an alicyclic dicarboxylic acid derivative represented by the following general formula (I):

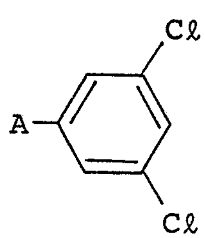

(I)

or a pharmacologically acceptable salt thereof, wherein A is a group represented by the following general formula (II):

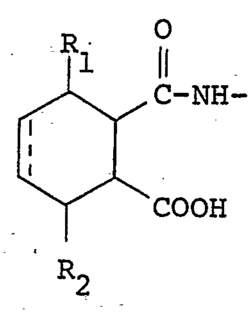

(II)

or general formula (III):

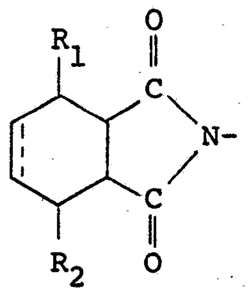

(III)

(in formulas (II) and (III), $R_1$ and $R_2$ represent a hydrogen atom or a lower alkyl group, provided that at least one of $R_1$ and $R_2$ represents a lower alkyl group, and --- represents a single bond or a double bond).

# INTERNATIONAL SEARCH REPORT

0302934

International Application No PCT/JP87/00121

**I. CLASSIFICATION OF SUBJECT MATTER** (if several classification symbols apply, indicate all) [3]

According to International Patent Classification (IPC) or to both National Classification and IPC

Int.Cl[4] C07C103/737, C07D209/48, A61K31/19, 31/40

**II. FIELDS SEARCHED**

Minimum Documentation Searched [4]

| Classification System | Classification Symbols |
|---|---|
| IPC | C07C103/737, C07D209/48, A61K31/19, 31/40 |

Documentation Searched other than Minimum Documentation to the Extent that such Documents are Included in the Fields Searched [5]

**III. DOCUMENTS CONSIDERED TO BE RELEVANT** [14]

| Category * | Citation of Document, [16] with indication, where appropriate, of the relevant passages [17] | Relevant to Claim No. [18] |
|---|---|---|
| A | JP, A, 59-231057 (Eisai Co., Ltd.) 25 December 1984 (25. 12. 84) Claim & DE, A1, 3421929 & GB, A1, 2141713 & FR, A1, 2548180 | 1-5, 6 |
| A | JP, A, 48-82038 (Takeda Chemical Industries, Ltd.) 2 November 1973 (02. 11. 73) Claim (Family: none) | 1-5 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| May 13, 1987 (13. 05. 87) | May 25, 1987 (25. 05. 87) |
| International Searching Authority [1] | Signature of Authorized Officer [20] |
| Japanese Patent Office | |